# EUROPÄISCHE PATENTSCHRIFT

(11) **EP 0 784 791 B1**
(45) Veröffentlichungstag und Bekanntmachung des Hinweises auf die Patenterteilung: **21.11.2001**
(21) Anmeldenummer: 95933316.2
(22) Anmeldetag: 30.09.1995
(51) Int. Cl.: G01N 33/543, G01N 27/414, C12Q 1/00

(54) **BIOSENSOR MIT AUF EINER SIGNALAKTIVEN OBERFLÄCHE KOVALENT FIXIERTEM BIOMATERIAL**
BIOSENSOR WITH BIOMATERIAL COVALENTLY FIXED ON A SIGNAL-ACTIVE SURFACE
BIODETECTEUR COMPORTANT UN BIOMATERIAU FIXE DE MANIERE COVALENTE SUR UNE SURFACE EMETTANT UN SIGNAL DE REACTION

(30) Priorität: 08.10.1994 DE 4436001
(43) Veröffentlichungstag der Anmeldung: 23.07.1997
(73) Patentinhaber: FORSCHUNGSZENTRUM JÜLICH GMBH, 52425 Jülich (DE)
(72) Erfinder: THUST, Marion, D-50939 Köln (DE); SCHÖNING, Michael, Josef, D-52428 Jülich (DE); VETTER, Joachim, D-50678 Köln (DE); KAUPP, Ulrich, Benjamin, D-52062 Aachen (DE); KORDOS, Peter, D-52428 Jülich (DE); LÜTH, Hans, D-52076 Aachen (DE)
(86) Internationale Anmeldenummer: DE9501371
(87) Internationale Veröffentlichungsnummer: WO9611402

(56) Entgegenhaltungen:
- EP-A- 0 127 438
- WO-A-88/08875
- US-A- 5 405 766
- US-A- 5 412 083
- JOURNAL OF PHYSICS E SCIENTIFIC INSTRUMENTS., Bd. 18, Nr. 9, September 1985 BRISTOL GB, Seiten 736-749, C. NYLANDER 'Chemical and biological sensors'

## Beschreibung

Die Erfindung bezieht sich auf Biosensoren, insbesondere auf dem Feldeffekt basierende Biosensoren, mit einer ggf. silanisierten signalaktiven Oberfläche, an die sensorisches Biomaterial mittels eines Crosslinkers gebunden ist.

Biosensoren, die für einen selektiven Nachweis bestimmter Analyten von enzymatischen Reaktionen Gebrauch machen, befinden sich bereits seit längerer Zeit in umfänglicher Entwicklung.

Grundsätzlich umfassen solche Sensoren eine Biomaterial, wie z.B. Enzym, enthaltende Oberflächenschicht, die dem zu überprüfenden Medium - insbesondere Flüssigkeit - ausgesetzt wird. Die unter Einwirkung des Analyten vom Biomaterial gelieferte Information wird von einem Transduktor-Element umgewandelt und mittels ggf. integrierter elektronischer Signalverarbeitung schließlich in registrierbarer Form erhalten. Als Transduktoren sind insbesondere potentiometrische und amperometrische Elektroden,wie Feldeffektstrukturen usw., im Gespräch.

Ein besonderes Problem solcher biomaterialhaltigen Sensoren ist die ausreichende, d.h. insbesondere bleibende, Fixierung bzw. Immobilisierung des Biomaterials an der Sensoroberfläche mit hoher flächenspezifischer Aktivität unter bestmöglicher Vermeidung einer Aktivitätsminderung durch die Art der Immobilisierung.

Als sensorisches Biomaterial kommen unterschiedliche Formen wie z.B. Enzyme, Mikroorganismen, Zellen, Antikörper, Antigene, Organellen oder Gewebeschnitte in Betracht. Vornehmlich finden sich in der einschlägigen Literatur jedoch Arbeiten über enzymhaltige Biosensoren, so daß im nachfolgenden vereinfachend enzymhaltige Biosensoren behandelt werden.

Als Immobilisierungstechniken sind die rein adsorptive Anlagerung, die Einbindung in Polymerschichten, die Vernetzung der Enzymmoleküle untereinander und die kovalente Anbindung der Enzyme an den Sensor bekannt. Für das kovalente Anbinden des Enzyms an die vorwiegend oxidische Sensorfläche wird diese üblicherweise silanisiert (meist mit einem Aminoalkylalkoxysilan) und anschließend mittels Glutaraldehyd für eine Ankopplung des Enzyms an die silanisierte Oberfläche gesorgt (siehe Anal.Chim.Acta 245 (1991) Seiten 89 - 99). Es wurde aber auch bereits eine Silanisierung von Si₃N₄-Oberflächen mit anschließender kovalenter Anbindung mittels unterschiedlicher Crosslinker beschrieben (siehe R.A. Williams et al., Biosensors & Bioelectronics 3 (1994) 159-67).

Daneben wurde auch die Verwendung von thiolterminierten Silanen empfohlen mit nachfolgender Protein-Immobilisierung mittels eines heterobifunktionellen Crosslinkers, wie z.B. mit N-Hydroxy-succinimidestern wie N-Succinimidyl-3-(2-pyridyldithio)-propionat (SPDS), N-Succinimidyl-p-jodacetylamido-benzoat (SIAB), N-Succinimidyl-4-(p-male-imido-phenyl)-butyrat (SMPB) oder N-Succinimidyl-m-maleimido-benzoat-derivaten (MBS-Derivaten) (siehe S.K. Bathia et al. Anal. Biochem., 178 (1989), 408-13).

Eine direkte Anbindung von Enzymen und Mikroorganismen an eine durch Plasmapolymerisation erzeugte Si₃N₄-Oberfläche wird in der JP 61 087 699 von 1986 beschrieben, wofür Glutaraldehyd oder Diisocyanat verwendet werden sollen. Die Reaktivität der funktionellen Gruppen sowohl der homo- oder bifunktionellen Crosslinker als auch der betroffenen Bindungspartner läßt Oligomerbildung und Diversifizierungen der Kopplungen zu, so daß die exakte kovalente Anbindung eines Biomoleküls über ein Crosslinker-Molekül an die signalaktive Oberfläche nicht gewährleistet erscheint.

Aus EP 0 127 438 A1 ist eine Diagnostiziervorrichtung bekannt, bei welcher mehrere biochemische Liganden an vorbestimmte Oberflächenbereiche eines Sensors gekoppelt sind. Dabei wird die unreaktive Siliziumoberfläche zunächst hydrolysiert und chloriniert und anschließend die entsprechenden Liganden mit Hilfe einer photoaktiven Funktion angekoppelt.

In WO 88/08875 wird ein Biochip Adsorber-Sensor offenbart, der Adsorber-Liganden über Bindungsproteine an die Sensoroberfläche koppelt, wobei die Bindungsproteine bei Hitze denaturieren und bei Kälte wieder renaturieren. Die Adsorber-Liganden werden durch Hitzebehandlung reversibel inaktiv und die Substanzen werden wieder freigesetzt, so daß dadurch eine Regenerierung des Sensors erfolgen kann.

Es wurde daher eine neue Art der Biomaterialimmobilisierung an Sensoroberflächen entwickelt, die zu eindeutigen Produkten mit hoher Sensitivität und Standzeit führt.

Die erfindungsgemäßen Biosensoren der eingangs genannten Art sind im wesentlichen dadurch gekennzeichnet, daß der Crosslinker durch einen heterobifunktionellen Crosslinker mit einer für die signalaktive Oberfläche spezifisch reaktiven Gruppe (A) einerseits und einer photoaktivierbaren biomaterial-reaktiven Gruppe (B) andererseits gebildet wird.

Mit einem solchen heterobifunktionellen Crosslinker, dessen biomaterial-reaktive Gruppe erst durch Photoaktivierung zur Reaktion gebracht wird, kann eine eindeutige Kopplung des Biomaterials an der Oberfläche erreicht werden, indem zunächst unter Vermeidung der Photoaktivierung eine Umsetzung des Crosslinkers mit der Oberfläche über eine entsprechend reaktive Gruppe vorgenommen wird. Nach Entfernung überschüssiger (nichtgebundener) Crosslinker-Reste erfolgt dann die Umsetzung mit Biomaterial unter entsprechender Belichtung.

Als Oberflächen kommen insbesondere Halbleitermaterialien wie Germanium oder Silicium sowie oxidische Materialien wie Siliciumoxid, Aluminiumoxid, Tantaloxid u. dgl. in reaktiver Form oder silanisiert in Frage, wobei insbesondere Mercapto- oder Aminogruppen aufweisende Silanisierungsschichten, z.B.auf Siliciumdioxid aufgebracht werden. Zweckmäßig ist auch eine Si₃N₄-Oberflächenschicht mit reaktiven Gruppen, insbesondere NHₓ- Gruppen, die für eine Kopplung insbesondere mit Aldehydgruppen, Estergruppen oder Imidoestergruppen zur Verfügung stehen.

Als photoaktivierbare, mit Biomaterial umsetzbare Gruppen eignen sich insbesondere alicyclisch oder aromatisch gebundene Nitro- oder Azidogruppen, die mit proteinischem Material unter Belichtung reagieren. Bekannt sind diesbezüglich Azidophenylreste oder Azidosalicylreste als Gruppe B enthaltende Crosslinker mit N-Hydroxy-succinimidoester-Gruppe als Gruppe A. Solche Crosslinker sind im Handel erhältlich (s. z.B. "immunoTechnology Catalog & Handbook" der Fa. Pierce von 1992/3; E 34-46).

Die photoaktivierte Umsetzung einer Azidophenylverbindung mit Protein unter Lichteinstrahlung der Wellenlänge 265-275 nm erfolgt z.B. nach der Gleichung:

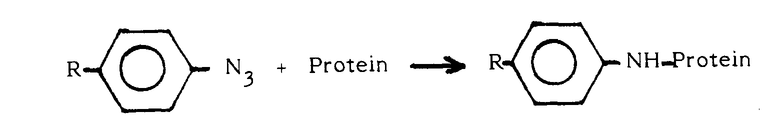

Als R eignen sich auf Grund ihrer Verfügbarkeit insbesondere Reste mit einer N-Hydroxysuccinimidoestergruppe als endständige Gruppe A, die mit NHₓ-Gruppen in folgender Weise reagieren kann

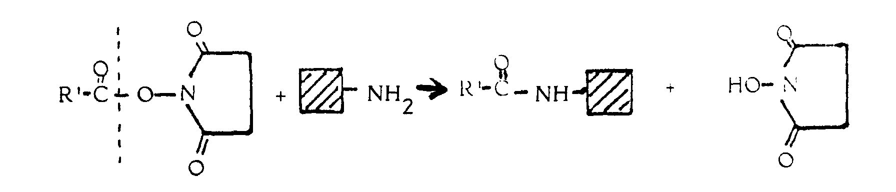

Als mit SH-Gruppen reagierende Funktionen A des Crosslinkers sind Disulfidgruppen bekannt.

Die Bildung von Siliciumnitridoberflächen kann insbesondere durch Abscheidung in CVD Technik aus einem SiH₄/NH₃-Gemisch erreicht werden (siehe A. Garde et al. in ESSDERC 1994- 11.-15. Sept. 1994 Ed. C. Hill & P. Ashburn). Sie nehmen an Luft Sauerstoff auf und neigen bei Einwirkung von Feuchtigkeit zur Hydrolyse unter Bildung von Si-OH, Si-NH und Si-NH₂ Gruppen. Diese mit der Gruppe A des Crosslinkers reaktiven Gruppen können für die Ankupplung von Biomaterial an die Nitridoberfläche ausgenutzt werden.

Besonders zweckmäßig wird bei der Realisierung der erfindungsgemäßen Sensoren eine durch Behandlung mit verdünnter Flußsäure von Oxiden befreite Si₃N₄-Oberfläche benutzt, mit deren NH₂-Gruppen oder auch NH-Gruppen ein Crosslinker reagiert, dessen photoaktivierbare Funktion B nachfolgend mit dem Protein zur Reaktion gebracht wird.

Als NH₂-reaktive Gruppen A des Crosslinkers bieten sich neben den bereits genannten auch Halogenid-, Epoxid-, Imid- oder Isocyanatfunktionen an. Eine Vielzahl von Reaktionsmöglichkeiten mit Aminogruppen findet sich im übrigen z.B. in der US-PS 5 234 820.

Die nachfolgend im einzelnen angegebenen aminospezifischen Reaktionen laufen bei Raumtemperatur in neutralem bis leicht alkalischem pH-Wert ab. Erhöhung sowohl der Temperatur als auch des pH-Werts steigert die Reaktionsgeschwindigkeit, jedoch auch die Hydrolyserate des Crosslinkers. Der benutzte Puffer sollte weder Amine noch andere Verbindungen enthalten, mit denen die funktionellen Gruppen des Crosslinkers reagieren könnten.

N-Hydroxysuccinimidester reagiert insbesondere spezifisch mit primären Aminen. Unter Abspaltung von N-Hydroxysuccinimid entsteht eine Amidbindung zwischen dem primären Amin und der Restgruppe des verwendeten Esters. Sofern kein wasserlösliches Analogon verwendet wird, muß ein Crosslinker, der diese funktionelle Gruppe besitzt zuerst in einer kleinen Menge eines organischen Lösungsmittels (z.B. DMSO) gelöst und erst dann auf die Endkonzentration in wäßrigem Puffer verdünnt werden. Die lonenstärke des Puffers sollte nicht zu hoch sein um Aussalzungseffekte zu vermeiden. Ein leicht alkalischer pH-Wert (7-9) garantiert, daß sich die primären Amine in unproptoniertem Zustand befinden.

Aldehyde verfügen über eine stark reduzierende Carbonylgruppe. Diese reagiert mit primären Aminen unter Wasserabspaltung.

Die Reaktion von primären Aminen mit Imidoestern läuft im pH-Bereich zwischen 8 und 9 ab. Der Ester wird dabei abgespalten und das primäre Amin bildet mit der Imidogruppe eine Guanidinoverbindung.

Besitzt der Crosslinker als funktionelles Ende A eine thiolspezifische Gruppe wie Maleimid, aktiviertes Halogenid oder Pyridyldisulfid, so muß die anzukoppelnde Oberfläche eine freie Sulfhydrylgruppe (überlicherweise eine Mercaptoalkylgruppe eines Alkoxysilans) besitzen.Um eine Oxidation dieser Gruppen zu vermeiden, muß der verwendete Puffer entgast werden.

Maleimid reagiert in leicht saurem bis neutralem Milieu (pH 6,5 - 7,5), während für Halogenide und Pyridylsulfid pH-Werte größer oder gleich 7 zu empfehlen sind.

Die Erfindung ist für die Anbindung von Biomaterial, insbesondere proteinischem Biomaterial, speziell unterschiedlichster Enzyme wie Hydrolasen, Oxidoreduktasen, Transferasen, Lyasen, Isomerasen und Ligasen bzw. Synthetasen, insbesonderen Penicillinase, Urease, Glukoseoxidase, Urokinase, Acetylcholinesterase, Butyrylcholinesterase oder Lactatoxidase für Biosensoren brauchbar. Sie wurde speziell am Beispiel von Penicillinsensoren getestet, weshalb sich die nachfolgende Beschreibung auf solche bezieht. Dabei wird auf die angefügten Bezeichnungen Bezug genommen. Diese zeigen schematisch:
- Fig. 1: ein Sensorprinzip (Meßanordnung),
- Fig. 2: eine typische Meßkurve für einen Konzentrationsbereich von 10⁻⁴ bis 10⁻¹ Mol/l Penicillin
- Fig. 3: die Kalibrierungskurve eines erfindungsgemäßen Sensors.

### Beispiel:

Auf p- bzw. p⁺-dotierten Siliciumwafern (1 mOhm.cm - 30 Ohm.cm) wurde zuerst eine elektrisch nicht leitende Schicht von Siliciumdioxid mit einer Dicke von 5 - 100 nm durch thermische Trockenoxidation zwischen 700 und 1200 °C (hier bei 1000 °C) in einem Diffusionsofen erzeugt. Darauf wurde ebenfalls nicht leitendes Siliciumnitrid mit einer Dicke von 10-100 nm durch chemische Abscheidung in der Gasphase (PECVD) aufgebracht. Das Verhältnis SiH₄/NH₃ im Reaktionsgas betrug 2/1, die Substrattemperatur 200 - 500 °C (hier 300 °C) und der Druck während der Abscheidung 1,3 - 4,0 mbar (1 - 3 Torr), hier 1,9 mbar (1,5 Torr). Es folgte ein Temperschritt unter N₂-Atmosphäre (5 - 60 Minuten bei 700 - 1000 °C). Schließlich wurde die unpolierte Substratseite mit einem Ohmschen Kontakt (z.B. 10 - 1000 nm A1, Au) versehen. Das verwendete Material wurde durch thermisches Aufdampfen im Vakuum bei einem Basisdruck <10⁻³ mbar aufgebracht. Die Abscheidungsrate lag zwischen 0,1 und 10 nm/s. Anschließend wurde der Wafer in einem RTA-Ofen bei 150 - 500 °C (hier 400 °C) in N₂-Atmosphäre getempert.

Unmittelbar vor Beginn des Enzymimmobilisierungsprozesses wurden die Wafer in Aceton, 2-Propanol und destilliertem Wasser im Ultraschallbad gereinigt und 10 - 60 s (hier 30 s) in verdünnter Flußsäure (1-10% HF) geätzt.

Bei Verwendung des heterobifunktionellen Crosslinkers ANB-NOS (N-5-Azido-2-nitrobenzoyloxysuccinimide) wurde dieser zunächst in einer kleinen Menge DMSO gelöst und dann mit 0,2 M Triethanolaminpuffer (pH 5-9) auf eine Endkonzentration von 0,5-10 mM verdünnt. Diese Lösung wurde auf die Si₃N₄-Oberfläche aufgebracht und zwischen 5 und 40 Minuten lang bei Raumtemperatur inkubiert. Bei niedrigerer Temperatur muß länger inkubiert werden. Nichtan die Siliciumnitridoberfläche gebundene Moleküle wurden durch Abspülen mit Triethanolaminpuffer (TEA) entfernt. Danach wurde das Enzym (Penicillinase, Typ 1 aus Bacillus Cereus, Sigma P 0389) in einem Puffer, der keine Aminogruppen enthält (z.B. TEA, insbesondere nicht TRIS- oder Glycinpuffer) gelöst (1000-5000 Units/ml) und auf die mit Crosslinker vorbehandelte Siliciumnitridoberfläche gegeben. Nach einer Inkubationszeit von 1 - 240 min (hier 15 min) bei Temperaturen zwischen 4 und 60 °C, insbesondere bei Raumtemperatur wurde die Anbindung der Enzymmoleküle an die noch freie funktionelle Gruppe des Crosslinkers durch Licht im Wellenlängenbereich von 320-350 nm induziert. Nach Abschluß des Immobilisierungsverfahrens wurden die einsatzfähigen Penicillinsensoren mit 0,1 M TRIS-Puffer (pH 7-8) und destilliertem Wasser gespült und mindestens 10 Minuten an Luft bzw. unter N₂- oder Inertgasatmosphäre getrocknet.

Mit den auf diese Weise hergestellten Feldeffektsensoren wurden Messungen zur Bestimmung der Penicillinkonzentration in wäßrigen Lösungen durchgeführt.

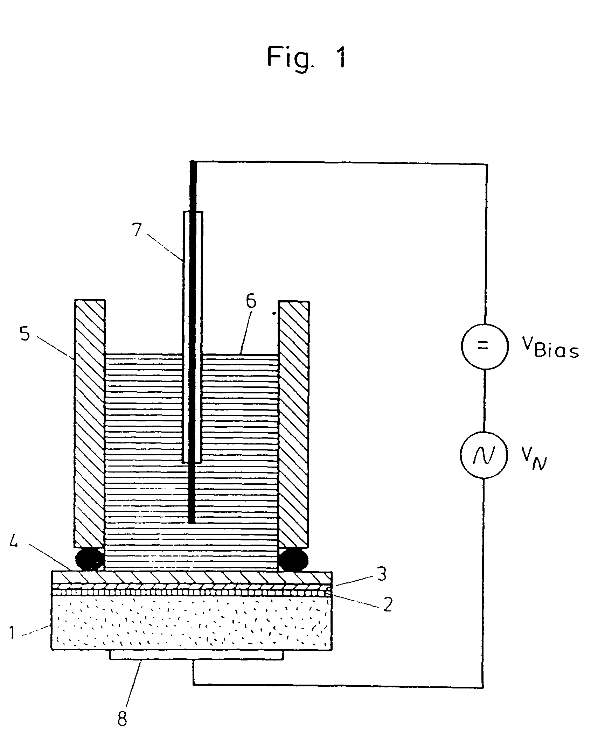

In **Fig. 1** ist die Meßanordnung schematisch dargestellt. Der erfindungsgemäß hergestellte Feldeffektsensor bestehend aus dem Siliciumsubstrat 1, der Isolatorschicht 2 (Siliciumdioxid und Siliciumnitrid), der Crosslinkerschicht 3 und der Enzymschicht (Penicillinschicht) 4 wurden in eine Meßzelle integriert. Die Meßzelle wurde mit einer wäßrigen Meßlösung befüllt, die Penicillin G in einer Konzentration zwischen 10⁻⁵ und 1 Mol/l enthielt. In die Meßlösung 6 taucht eine Referenzelektrode (z.B. Ag/AgCl) 7 ein. Die Potentiale werden über die Referenzelektrode 7 und eine Kontaktelektrode 8 am Siliciumsubstrat abgegriffen.

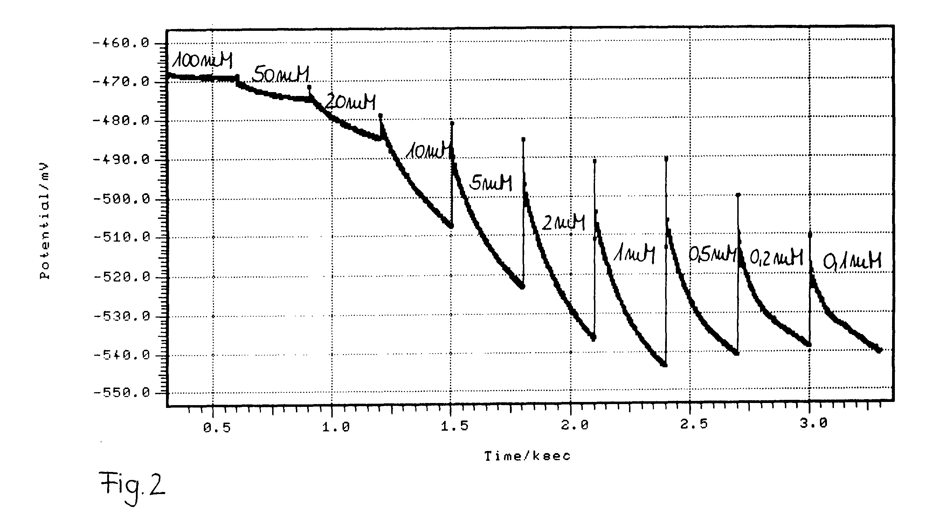

In **Fig. 2** ist eine typische Meßkurve dargestellt, die im CONCAP (CONstant CAPacitance)-Modus im Konzentrationsbereich zwischen 10⁻⁴ und 10⁻¹ Mol/l Penicillin aufgenommen wurde. Penicillin G Natriumsalz (Sigma P 3032) wurde dazu in 10 mM TRIS-HCI Puffer, pH 7 gelöst. Mit steigender Penicillinkonzentration steigt die Konzentration der gebildeten Penicilloinsäure und somit die Konzentration der Wasserstoffionen in unmittelbarer Nähe der als pH-Transduktor wirkenden Siliciumnitridfläche. Dies hat eine Verschiebung des Potentials an der Grenzfläche Siliciumnitrid/Elektolyt zu positiveren bzw. negativeren Spannungswerten hin zur Folge. Die Auftragung über der Zeit erlaubt eine Beobachtung des konzentrationsabhängigen Potentialverlaufs der Enzymreaktion. Zu gekennzeichneten Zeiten fand der Wechsel der Meßlösungen statt.

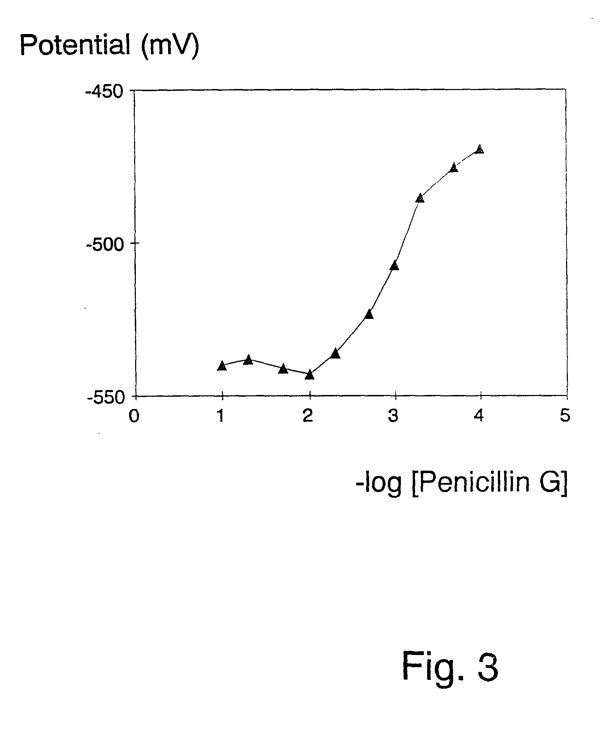

**Fig. 3** zeigt die aus **Fig. 2** ermittelte chemische Übertragungskennlinie. Sie stellt die Kalibrierungskurve des erfindungsgemäß hergestellten Feldeffektsensors dar. Angaben über die Sensitivität eines potentiometrischen Chemo- oder Biosensores werden im Hinblick auf den zugrundeliegenden Nernstschen Zusammenhang im linearen Bereich dieser Kurve, d.h. in dem Bereich, in dem ein logarithmischer Zusammenhang zwischen der Penicillinkonzentration und dem anliegenden Potential besteht, gemacht. Dieser Bereich liegt im erfindungsgemäß hergestellten Sensor zwischen pPenicillin G 2,3 und 3,3, was 0,5 und 5 mM entspricht. Die Empfindlichkeit beträgt 50 mV pro Dekade.

Die exakte Lage des linearen Meßbereichs und die absolute Empfindlichkeit hängen wesentlich von der Wahl der Pufferzusammensetzung, dessen Konzentration, d.h. der Pufferkapazität und dem pH-Wert ab. Durch geeignete Wahl dieser Parameter kann der für eine Messung erforderlich Meßbereich gezielt eingestellt werden. Beispielsweise liegt der lineare Meßbereich bei Verwendung von IMIDAZOL-Puffer (pH 7) zwischen 2 und 20 mM Penicillin, für HEPES-Puffer etwa zwischen 1 und 10 mM. Erhöhung des pH-Werts verschiebt die Lage der linearen Bereiche der Kalibrationskurven zu höheren Penicillinkonzentrationen hin, Erniedrigung entsprechend zu niedrigeren.

Die erfindungsgemäß hergestellten Sensoren weisen eine hohe Langzeitstabilität von mehr als 250 Tagen auf. Die Empfindlichkeit liegt bei 50 mV pro Penicillindekade.

Die Herstellung eines Feldeffekttransitors, der im Gatebereich einen gleichartigen Aufbau wie die in der Erfindung beschriebene kapazitive Schichtstruktur aufweist, ist möglich.

## Patentansprüche

1. Verfahren zur Herstellung eines Biosensors, der eine Oberfläche aufweist und dessen sensorisches Biomaterial mittels eines Crosslinkers kovalent an die Oberfläche gebunden ist,
**dadurch gekennzeichnet, daß**
- ein Sensor mit einer Si₃N₄-Oberfläche mit reaktiven -Si-NH oder -Si-NH₂ Gruppen ausgewählt wird,
- diese Oberfläche direkt mit einem heterobifunktionellen Crosslinker in Kontakt gebracht wird, wobei die oberflächenspezifisch-reaktive Gruppe (A) des Crosslinkers direkt an die reaktiven Gruppen der Si₃N₄-Oberfläche koppelt, und
- die photoaktivierbare Biomaterial-reaktive Gruppe (B) des Crosslinkers durch Photoaktivierung mit dem sensorischen Biomaterial zur Reaktion gebracht wird.

2. Verfahren zur Herstellung eines Biosensors nach Anspruch 1,
**dadurch gekennzeichnet,**
**daß** die photoaktivierbare Biomaterial-reaktive Gruppe (B) des Crosslinkers eine Azido- oder eine Nitro-Gruppe ist.

3. Verfahren zur Herstellung eines Biosensors nach einem der vorhergehenden Ansprüche 1-2,
**dadurch gekennzeichnet,**
**daß** die photoaktivierbare Biomaterial-reaktive Gruppe (B) des Crosslinkers eine aromatisch gebundene Azido- oder eine Nitro-Gruppe ist.

4. Verfahren zur Herstellung eines Biosensors nach einem der vorhergehenden Ansprüche 1-3,
**dadurch gekennzeichnet,**
**daß** die Oberflächen spezifische reaktive Gruppe (A) des Crosslinkers eine Aldehyd-, Ester- oder Imidoestergruppe ist.

5. Verfahren zur Herstellung eines Biosensors nach einem der vorhergehenden Ansprüche 1-4,
**dadurch gekennzeichnet,**
**daß** die Oberflächen spezifische reaktive Gruppe (A) des Crosslinkers eine N-Hydroxy-succinimidester-Gruppe ist.

6. Verfahren zur Herstellung eines Biosensors nach einem der vorhergehenden Ansprüche,
**dadurch gekennzeichnet,**
**daß** die Oberfläche eine 10-1000 nm dicke Si₃N₄-Schicht ist, die durch Abscheidung in CVD (chemical vapor deposition)-Technik aus einer SiH₄/NH₃-Mischung auf einer SiO₂-Fläche hergestellt wurde.

## Claims

1. A method of producing a biosensor which comprises a surface and the biomaterial sensor of which is covalently bonded to the surface by means of a crosslinker,
**characterised in that**
- a sensor is selected which has an Si₃N₄ surface comprising reactive -Si-NH or -Si-NH₂ groups,
- said surface is brought directly into contact with a heterobifunctional crosslinker, wherein the surface-specific reactive group (A) of the crosslinker couples directly to the reactive groups of the Si₃N₄ surface, and
- the photo-activatable biomaterial-reactive group (B) of the crosslinker is caused to react with the biomaterial sensor by photo-activation.

2. A method of producing a biosensor according to claim 1,
**characterised in that**
the photo-activatable biomaterial-reactive group (B) of the crosslinker is an azido or nitro group.

3. A method of producing a biosensor according to either one of the preceding claims 1-2,
**characterised in that**
the photo-activatable, biomaterial-reactive group (B) of the crosslinker is an aromatically bonded azido or nitro group.

4. A method of producing of a biosensor according to any one of the preceding claims 1-3,
**characterised in that**
the surface-specific reactive group (A) of the crosslinker is an aldehyde, ester or imidoester group.

5. A method of producing a biosensor according to any one of the preceding claims 1-4,
**characterised in that**
the surface-specific reactive group (A) of the crosslinker is an N-hydroxysuccinimide ester group.

6. A method of producing a biosensor according to any one of the preceding claims,
**characterised in that**
the surface is an Si₃N₄ layer which has a thickness of 10-1000 nm and which is produced from an SiH₄/NH₃ mixture by deposition on to an SiO₂ surface by a CVD (chemical vapour deposition) technique.

## Revendications

1. Procédé de fabrication d'un biodétecteur qui comporte une surface et dont le biomatériau de détection est lié au moyen d'un réticulant de manière covalente à la surface,
**caractérisé en ce que**
- on choisit un détecteur ayant une surface en Si₃N₄ à groupes -Si-NH ou -Si-NH₂ réactifs,
- on met cette surface directement en contact avec un réticulant hétérobifonctionnel, le groupe (A) réactif spécifiquement à la surface du réticulant se couplant directement aux groupes réactifs de la surface en Si₃N₄, et
- on met le groupe (B) réactif au biomatériau et pouvant être photoactivé du réticulant à réagir par photoactivation sur le biomatériau de détection.

2. Procédé de fabrication d'un biodétecteur suivant la revendication 1,
**caractérisé**
**en ce que** le groupe (B) réactif au biomatériau et pouvant être photoactivé du réticulant est un groupe azido ou un groupe nitro.

3. Procédé de fabrication d'un biodétecteur suivant l'une des revendications précédentes 1 ou 2, **caractérisé en ce que** le groupe (B) réactif au biomatériau ou qui peut être photoactivé du réticulant est un groupe azido ou un groupe nitro lié de manière aromatique.

4. Procédé de fabrication d'un biodétecteur suivant l'une des revendications précédentes 1 à 3, **caractérisé en ce que** le groupe (A) réactif d'une manière spécifique à la surface du réticulant est un groupe aldéhyde, un groupe ester ou un groupe imidoester.

5. Procédé de fabrication d'un biodétecteur suivant l'une des revendications précédentes 1 à 4, **caractérisé**
**en ce que** le groupe (A) réactif spécifiquement à la surface du réticulant est un groupe N-hydroxysuccinimide-ester.

6. Procédé de fabrication d'un biodétecteur suivant l'une des revendications précédentes, **caractérisé en ce que** la surface est une couche de Si₃N₄ de 10 à 100 mm d'épaisseur qui a été préparée par dépôt par une technique CVD (chemical vapor deposition) à partir d'un mélange de SiH₄/NH₃ sur une surface en SiO₂.
